(19) Europäisches Patentamt
European Patent Office
Office européen des brevets

(11) Veröffentlichungsnummer: **0 575 425 B1**

(12) # EUROPÄISCHE PATENTSCHRIFT

(45) Veröffentlichungstag der Patentschrift: **11.10.95**

(51) Int. Cl.6: **A61K 31/415**, A61K 31/42, C07D 487/04, C07D 498/04, //(C07D487/04,235:00,209:00), (C07D498/04,263:00,209:00)

(21) Anmeldenummer: **92906522.5**

(22) Anmeldetag: **13.03.92**

(86) Internationale Anmeldenummer: **PCT/EP92/00558**

(87) Internationale Veröffentlichungsnummer: **WO 92/16207 (01.10.92 92/25)**

(54) **VERWENDUNG VON OXAZOLO- 2,3-a]ISOINDOL- UND IMIDAZO 2,1-a]ISOINDOL-DERIVATEN ALS ANTIVIRALE ARZNEIMITTEL SOWIE NEUE OXAZOLO 2,3-a]ISOINDOL-DERIVATEN.**

(30) Priorität: **15.03.91 DE 4108395**

(43) Veröffentlichungstag der Anmeldung: **29.12.93 Patentblatt 93/52**

(45) Bekanntmachung des Hinweises auf die Patenterteilung: **11.10.95 Patentblatt 95/41**

(84) Benannte Vertragsstaaten: **AT BE CH DE DK ES FR GB GR IT LI LU NL SE**

(56) Entgegenhaltungen:
**EP-A- 0 121 776**
**US-A- 3 334 113**
**US-A- 3 336 306**

(73) Patentinhaber: **BOEHRINGER MANNHEIM GMBH**

**D-68298 Mannheim (DE)**

(72) Erfinder: **KÖNIG, Bernhard**
**Dürrbergstr. 28**
**D-8137 Berg (DE)**
Erfinder: **LESER, Ulrike**
**Stiftsbogen 64**
**D-8000 München 70 (DE)**
Erfinder: **MERTENS, Alfred**
**Beethovenstr. 20**
**D-6905 Schriesheim (DE)**
Erfinder: **SCHÄFER, Wolfgang**
**Feldbergstr. 60**
**D-6800 Mannheim 1 (DE)**
Erfinder: **POLL, Thomas**
**Gambrinusstr. 4 A**
**D-6800 Mannheim 31 (DE)**

(74) Vertreter: **Mink, Reinhold, Dr. et al**
**c/o Boehringer Mannheim GmbH,**
**Patentabteilung,**
**Sandhoferstrasse 116**
**D-68298 Mannheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegenden Erfindung betrifft die Verwendung von Oxazolo[2,3-a]isoindol- und Imidazo[2,1-a]-isoindol-Derivaten als antivirale Arzneimittel sowie neue optisch aktive Derivate und neue Oxazolo[2,3-a]-isoindol-Derivate, Verfahren zu deren Herstellung und Arzneimittel, die diese Verbindungen enthalten.

Die Verwendung von Oxazolo[2,3-a]isoindol- und Imidazo[2,1-a]isoindol-Derivaten als Arzneimittel ist in mehreren Publikationen beschrieben. So werden Derivate dieser Substanzklassen in J.Org.Chem. 55, 3088, 1990 als Inhibitoren der gamma-Butyrobetain-hydroxylase beschrieben. Weiterhin sind folgende pharmakologische Wirkungen beschrieben:

a) Appetit Suppressor Wirkung in US 3,994,920 und US 3,935,218,
b) Behandlung von Gastritis in US 3,966,955,
c) Antidepressive Wirkung in US 3,935,218, US 3,900,494, US 3,898,226, US 3,898,231, US 3,885,037, US 3,867,394, US 3,867,394 und US 3,763,178,
d) Diuretische Wirkung in US 3,935,218, US 3,898,226, US 3,898,231, US 3,885,037 und US 3,867,394,
e) Antihyperglycemische Wirkung in US 3,928,597,
f) Anorexische Wirkung in US 3,898,226, US 3,898,231 und US 3,885,037,
g) Antiinflammatorische Wirkung in CH 480350 und US 3,408,350,
h) Analgetische Wirkung in CH 480,350, CH 482,697, CH 481,124 und CH 481,123,
i) Blutdrucksenkende Wirkung in CH 480,350, CH 481,124 und CH 481,123
j) Spasmolytische Wirkung in CH 480,350, CH 481,124 und CH 481,123,
k) Tranquilizer und sedative Wirkung in CH 480,350 und CH 481,123,
l) Antitussive Wirkung in CH 480,350, CH 481,124 und CH 481,123 und
m) Rheumatische Wirkung in CH 482,697.

Die Oxazolo[2,3-a]isoindol- und Imidazo[2,1-a]isoindol-Derivate der allgemeinen Formel I besitzen teilweise auch ein gewisses Potential als Zwischenprodukte zur Herstellung von strukturell ähnlichen Verbindungsklassen. Diese Zwischenprodukte sind beschrieben in CS 201,499; Aust. J.Chem., 35, 2307, 1982; US 4,018,765; GB 1,225,411; US 3,925,359; US 3,929,766; US 3,910,947; US 3,905,994; J. Med. Chem. 18, 177, 1975; J. Org. Chem. 40, 382, 1975; DE 1,795,785; GB 1,322,339; US 3,663,532; GB 1,258,946; FR 7457; DE 2,106,694; GB 1,225,411; GB 1,232,469; GB 1,225,413; FR 1,580,180; FR 1,580,184; FR 1,571,331; US 3,454,592; US 3,441,572; ZA 6,801,724; J. Org. Chem. 34, 1720, 1969; ZA 6,801,872; US 3,379,733.

Die Synthese der Verbindungen der allgemeinen Formel I ist u.a. beschrieben in J. Heterocycl. Chem. 26, 1441, 1989; Gazz. Chim. Ital. 155 (12, Pt.B), 653, 1985; Bull. Soc. Chim. Belg. 95, 197, 1986; J. Chem. Soc., Perkin Trans. 1, 809, 1985; J. Org. Chem. 45, 4049, 1980; US 3,867,401; DE 2,332,232; US 3,657,221; US 3,507,863; GB 1,059,175; J. Org. Chem. 34, 165, 1969; US 3,403,164; J. Org. Chem. 33, 2874, 1968; US 3,336,306; US 3,334,113; NL 6,613,264; J. Org. Chem. 32, 2180, 1967; J. Org. Chem. 32, 2185, 1967 und Belg. 659,530.

Die Erfindung betrifft die Verwendung von Oxazolo[2,3-a]-isoindol- und Imidazo[2,1-a]isoindol-Derivate der allgemeinen Formel I

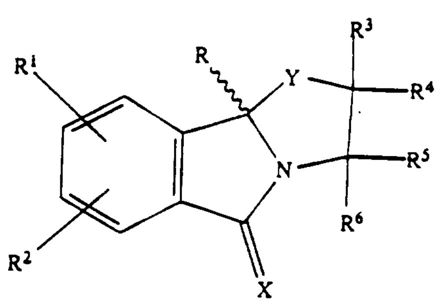

(I),

zur Herstellung von Arzneimitteln mit antiviraler Wirkung, wobei

X ein Sauerstoff- oder Schwefelatom, die Iminogruppe =NH oder eine $=N-C_1-C_5$-Alkyliminogruppe sein kann,

Y ein Sauerstoffatom oder die Gruppe $NR^7$ sein kann, wobei $R^7$ ein Wasserstoffatom oder einen $C_1-C_6$-Alkyl- oder $C_1-C_6$-Acylrest bedeutet,

R ein Wasserstoffatom, einen geradkettigen oder verzweigten, gesättigten oder ungesättigten aliphatischen Rest mit 1-9 C-Atomen, der durch Phenyl substituiert sein kann, oder

einen $C_1$-$C_6$-Alkoxy-$C_1$-$C_6$-alkyl- oder $C_1$-$C_6$-Alkylmercapto-$C_1$-$C_6$-alkylrest bedeutet, oder einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylamino-carbonyl, $C_1$-$C_6$-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl, oder

einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind,

$R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Trifluormethyl-, Carboxy, Halogen, Hydroxy, Nitro, Cyano, Azido, Phenyl oder Benzyloxy bedeutet,

$R^2$ die gleiche Bedeutung hat wie $R^1$, wobei die Reste $R^1$ und $R^2$ unabhängig voneinander gleich oder verschieden sein können,

$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Halogen, Cyano, Hydroxy, Carboxy, $C_1$-$C_6$-Alkoxycarbonyl, Aminocarbonyl, $C_1$-$C_6$-Alkylaminocarbonyl oder Di-$C_1$-$C_6$-alkylaminocarbonyl bedeutet,

$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,

sowie deren Tautomere, Enantiomere, Diastereomere und physiologisch verträgliche Salze.

Für den Fall, daß Y ein Sauerstoffatom ist, $R^1$ und $R^2$ nicht gleichzeitig Wasserstoffatome bedeuten und $R_1$ oder $R_2$ nicht wiederes Alkyl, Alkoxy, Amino, Halogen, Nitro und Trifluormethyl bedeuten, handelt es sich um neue Oxazolo-[2,3-a]isoindol-Derivate, die ebenfalls Gegenstand der vorliegenden Erfindung sind.

Die Verbindungen der Formel I sind bisher nur in Form ihrer Racemate bekannt. Es hat sich nun gezeigt, daS die optisch aktiven Derivate eine höhere Wirksamkeit besitzen als die entsprechenden racemischen Gemische, so daS die vorliegende Erfindung sich auch auf die neuen R- und S-Enantiomeren bezieht.

Die Verbindungen der Formel I weisen wertvolle pharmakologische Eigenschaften auf. Insbesondere eignen Sie sich zur Therapie und Prophylaxe von Infektionen, die durch DNA-Viren wie z.B. das Herpes-Simplex-Virus, das Zytomegalie-Virus, PapillomaViren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Die Verbindungen der allgemeinen Formel I besitzen eine ausgeprägte antivirale Wirkung und eignen sich insbesondere zur Behandlung von viralen bzw. retro-viralen Infektionen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Aquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir$^R$, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Es konnte nun nachgewiesen werden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutica besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

Die Trennung der Racemate in die Enantiomeren kann analytisch, semipräparativ und präparativ chromatographisch auf geeigneten optisch aktiven Phasen mit gängigen Elutionsmitteln durchgeführt werden.

Als optisch aktive Phasen eignen sich beispielsweise optisch aktive Polyacrylamide oder Polymethacrylamide, z.T. auch an Kieselgel (z.B. ChiraSpher (R) von Merck, Chiralpak (R) OT/OP von Baker), Celluloseester/-carbamate (z.B. Chiracel (R) OB/OY von Baker/Daicel), Phasen auf Cyclodextrin- oder Kronenetherbasis (z.B. Crownpak (R) von Daicel) oder mikrokristallines Cellulosetriacetat (Merck).

Ein aliphatischer Rest bedeutet einen geradkettigen oder verzweigten Alkyl-, Alkenyl- oder Alkinylrest mit 1-9, vorzugsweise 2-7 Kohlenstoffatomen, wie z.B. der Propyl-, Isopropyl-, Butyl-, Isobutyl-, Pentyl-, Hexyl- oder Heptylrest. Als ungesättigte Reste kommen $C_2$-$C_7$-Alkenyl- und Alkinylreste in Frage, bevorzugt $C_2$-$C_5$, wie z.B. der Allyl-, Dimethylallyl-, Butenyl-, Isobutenyl-, Pentenyl- oder Propinylrest.

Ein aliphatischer Rest, der durch Phenyl substituiert sein kann, ist insbesondere eine Phenyl-$C_1$-$C_6$-alkylgruppe, wie z.B. der Benzyl-, Phenethyl-, Phenylpropyl- oder Phenylbutylrest.

Bedeutet R einen Phenylring, so kann dieser ein-, zwei- oder dreifach substituiert sein. Die Substituenten können unabhängig voneinander in o-, m- oder p-Stellung stehen.

Ein carbocyclischer Ring mit 7-15 C-Atomen kann mono-, bi- oder tricyclisch sein und pro Ring jeweils 5 oder 6 C-Atome aufweisen. Dieser Ring kann gesättigt, ungesättigt, teilweise gesättigt oder aromatisch sein. Beispielhaft genannt seien die folgenden Ringsysteme: der Naphthyl-, Anthracenyl-, Phenanthrenyl-, Flourenyl-, Indenyl-, Indanyl-, Acenaphthylenyl-, Norbornyl-, Adamantylring oder eine $C_3$-$C_7$-Cycloalkyl- oder $C_5$-$C_8$-Cycloalkenylgruppe.

Die heterocylischen mono-, bi- oder tricyclischen Ringsysteme enthalten pro Ringsystem 5 oder 6 Kohlenstoffatome, wobei 1-4 bzw. 1-5 C-Atome durch die Heteroatome Sauerstoff, Schwefel und/oder Stickstoff ersetzt sein können. Die Ringsysteme können aromatisch, partiell oder vollständig hydriert sein. Beispielhaft genannt seien die folgenden Ringsysteme: das Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei die ungesättigten bzw. aromatischen Carbo- und Heterocyclen partiell oder vollständig hydriert sein können.

R bedeutet bevorzugt unsubstituiertes Phenyl oder Phenyl einoder zweifach substituiert durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_3$-$C_6$-Alkenyloxy, $C_1$-$C_6$-Alkylamino, $C_1$-$C_6$-Dialkylamino-, $C_1$-$C_6$-Alkylcarbonylamino, $C_1$-$C_6$-Alkylaminocarbonyl, $C_1$-$C_6$-Alkoxycarbonyl-, Amino, Hydroxy, Nitro, Azido, Trifluormethyl, Cyano oder Halogen. Bevorzugt enthalten die zuvor genannten "Alkyl"-teile in den jeweiligen Definitionen bis zu 4, insbesondere bis zu 3 C-Atome.

Carbocylische Ringe sind bevorzugt Biphenyl, Naphthyl, Anthracenyl, Indenyl, Fluorenyl, Acenaphthylenyl, Phenanthrenyl, Norbornyl, Adamantyl, $C_3$-$C_6$-Cycloalkyl, $C_5$-$C_8$-Cycloalkenyl.

Heterocyclische Ringsysteme sind bevorzugt Pyrrol, Imidazol, Furan, Thiophen, Pyridin, Pyrimidin, Thiazol, Triazin, Indol, Chinolin, Isochinolin, Cumaron, Thionaphthen, Benzimidazol, Chinazolin, Methylendioxybenzol, Ethylendioxybenzol, Carbazol, Acridin und Phenothiazin.

Für die Reste $R^1$ und $R^2$ sind Wasserstoff, $C_1$-$C_6$-Alkyl, $C_2$-$C_6$-Alkenyl, $C_2$-$C_6$-Alkinyl, $C_1$-$C_6$-Alkoxy, $C_1$-$C_6$-Alkylmercapto, $C_1$-$C_6$-Alkylamino, $C_1$-$C_6$-Alkoxycarbonyl, Trifluormethyl, Amino, Halogen, Hydroxy, Cyano und Azido bevorzugt, wobei die "Alkyl"-teile in den zuvor genannten Definitionen bevorzugt bis zu 4, insbesondere bis zu 3 C-Atome enthalten.

Bevorzugte Substituenten für $R^3$, $R^4$, $R^5$ und $R^6$ sind Wasserstoff, $C_1$-$C_6$-Alkyl, $C_1$-$C_6$-Alkoxy, $C_1$-$C_6$-Alkylmercapto, Carboxy, $C_1$-$C_6$-Alkoxycarbonyl, Halogen, Cyano und Hydroxy, wobei die "Alkyl"-teile in den zuvor genannten Definitionen bevorzugt bis zu 4, insbesondere bis zu 3 C-Atome enthalten.

X ist bevorzugt Sauerstoff oder Schwefel. Unter Halogen ist allgemein Fluor, Chlor, Brom und Iod zu verstehen, bevorzugt Fluor, Chlor und Brom.

Y ist bevorzugt Sauerstoff oder -$NR^7$, wobei für $R^7$ Wasserstoff oder der $C_1$-$C_6$-Alkyl- oder $C_1$-$C_6$-Acylrest in Frage kommt. Unter Acylrest versteht man insbesondere den $C_1$-$C_6$-Alkylcarbonylrest. Bevorzugt enthalten die "Alkyl"-teile bis zu 4, insbesondere bis zu 3 C-Atome.

Besonders bevorzugte Reste für R sind $C_3$-$C_5$-Alkyl, Phenyl, durch $C_1$-$C_6$-Alkyl, $C_1$-$C_6$-Alkoxy, Trifluormethyl oder Halogen, mono- oder disubstituiertes Phenyl, Naphthyl, Anthracenyl, Indanyl, Furyl, Thienyl, Pyridyl, Indolyl, Chinolinyl.

Für $R^1$ und $R^2$ sind unabhängig voneinander besonders bevorzugt Wasserstoff, Methyl, Ethyl, Isopropyl, Trifluormethyl, Methoxy, Ethoxy, und Halogen, wobei Chlor und Brom für Halogen besonders bevorzugt sind.

Für $R^3$, $R^4$, $R^5$ und $R^6$ sind Aminocarbonyl, Methyl, Ethyl und Isopropyl besonders bevorzugt.

Insbesondere bevorzugt sind Verbindungen der allgemeinen Formel I, in denen R, $R^1$, X und Y die oben angegebene Bedeutung haben und $R^2$, $R^3$, $R^4$, $R^5$ und $R^6$ gleich Wasserstoff, Methyl, Ethyl, Chlor, Brom, Methoxy oder Ethoxy sind, wobei $R^2$ bis $R^6$ vor allem Wasserstoff darstellen.

Die Arzneimittel enthaltend mindestens eine Verbindung der Formel I zur Behandlung von viralen oder retroviralen Infektionen oder von durch diese verursachten Erkrankungen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Zur Herstellung von physiologisch verträglichen Salzen werden Verbindungen der Formel I, die eine basische Gruppe tragen, mit anorganischen oder organischen Säuren umgesetzt, wie z.B mit Salzsäure, Bromwasserstoffsäure, Schwefelsäure, Phosphorsäure, Fumarsäure, Bernsteinsäure, Weinsäure, Zitronensäure, Milchsäure oder Maleinsäure, und die Säureadditionssalze isoliert. Enthalten die Verbindungen der Formel I eine Säuregruppe, so erhält man die physiologisch verträglichen Salze durch Umsetzung mit Alkali- oder Erdalkalihydroxiden, wie z.B. Natriumhydroxid, Kaliumhydroxid oder Calciumhydroxid, oder mit anderen basischen Gruppen, wie Aminen, z.B. Triethylamin.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die Arzneimittel enthaltend mindestens eine Verbindung der Formel I werden dadurch hergestellt, indem man eine Verbindung der Formel I mit üblichen pharmazeutischen Hilfsstoffen mischt und zu Arzneiformen, wie z. B. Tabletten, Dragees, Kapseln oder Lösungen verarbeitet. Diese Arzneiformen werden zu verkaufsfertigen Verpackungseinheiten konfektioniert, und mit einem entsprechenden Hinweis, z. B. in Form eines Beipackzettels oder eines Verpackungsaufdruckes, versehen, aus dem die Verwendung zur Behandlung von viralen oder retroviralen Infektionen bzw. von durch diese Infektionen bedingten Erkrankungen hervorgeht.

Die erfindungsgemäßen Verbindungen der allgemeinen Formel I werden nach literaturbekannten Verfahren hergestellt, indem man gegebenenfalls substituierte Benzoesäurederivaten der allgemeinen Formel II

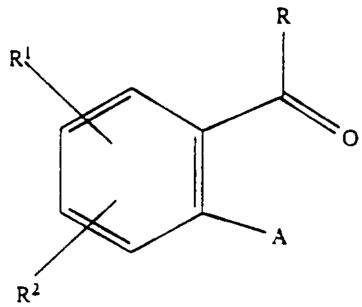

(II),

in der R, $R^1$, und $R^2$ die oben angegebene Bedeutung haben und A gleich -COOH oder C=N ist, mit substituiertem oder unsubstituiertem Ethanolamin oder Ethylendiamin der allgemeinen Formel III

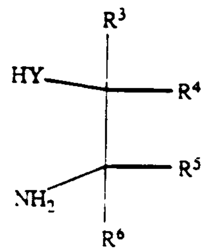

(III),

in der Y, $R^3$, $R^4$, $R^5$ und $R^6$ die oben angegebene Bedeutung haben, in einem geeigneten inerten Lösungsmittel bei Raumtemperatur bis Rückflußtemperatur evtl. in Gegenwart katalytischer Mengen Säure, z.B. p-Toluolsulfonsäure, umsetzt und gegebenenfalls anschließend Verbindungen der Formel I in andere Verbindungen der Formel I nachträglich umwandelt und anschließend chromatographisch bzw. durch Umkristallisation reinigt. Racemate können durch Chromatographie an geeigneten optisch aktiven Phasen, z.B. Cellulosetriacetat, in die Antipoden getrennt werden.

Die nachträglichen Umwandlungen von Verbindungen der Formel I in andere Verbindungen der Formel I betreffen die Herstellung von Oxazolo[2,3-a]isoindol- oder Imidazo[2,1-a]isoindol-Derivaten mit X=S oder N-Alkylimin. Verbindungen mit X=S werden hergestellt durch Umsetzung von Verbindungen der Formel I, in der X ein Sauerstoffatom bedeutet, mit schwefelgruppenübertragenden Verbindungen, wie z.B. Lawesson's Reagenz. Verbindungen mit X = N-Alkylimino werden hergestellt durch Umsetzung der entsprechenden Iminoverbindungen der allgemeinen Formel I mit Alkylaminen nach an sich bekannten Methoden.

Die Benzoesäurederivate der allgemeinen Formel II sind ebenfalls literaturbekannt und werden z.B. durch Friedel-Crafts-Acylierung von substituiertem oder unsubstituiertem Phthalsäureanhydrid mit gegebenenfalls substituierten Arenen in Gegenwart einer Lewis-Säure (z.B. Aluminiumchlorid) oder durch Reaktion von Grignardreagenzien der allgemeinen Formel IV

R-MgBr (IV),

in der R mit Ausnahme von Wasserstoff die oben angegebene Bedeutung hat, mit Phthalsäureanhydrid, das gegebenenfalls substituiert ist, in geeigneten inerten Lösungsmitteln bei tiefen Temperaturen hergestellt.

Die Verfahren zur Herstellung der erfindungsgemäßen Verbindungen der allgemeinen Formel I können auch der im Stand der Technik genannten Patentanmeldungen bzw. Literaturstellen entnommen werden.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die als racemischen Gemische oder in optisch aktiver Form bzw. als reine R- und S-Enantiomeren vorliegen können.

Verbindungen der Formel I, in der Y ein Sauerstoffatom bedeutet, sind insbesondere die folgenden:

1. 8,9b-Dimethyl-2,3-dihydrooxazolo-[2,3-a]isoindol-5(9bH)-on
2. 8-Chlor-9b-phenyl-2,3-dihydrooxazolo-[2,3-a]isoindol-5(9bH)-on
3. 8-Fluor-9b-(4-methylphenyl)-2,3-dihydrooxazolo-[2,3-a]isoindol-5(9bH)-on
4. 8-Chlor-9b-(3-methylphenyl)-2,3-dihydrooxazolo-[2,3-a]isoindol-5(9bH)-on
5. 3-Methyl-9b-(4-ethylphenyl)-2,3-dihydrooxazolo-[2,3-a]isoindol-5(9bH)-on

6. 9b-(2,3-Dimethylphenyl)-2,3-dihydrooxazolo-[2,3-a]isoindol-5(9bH)-thion
7. 8-Chlor-9b-(3,4-dimethylphenyl)-2,3-dihydrooxazolo-[2,3-a]isoindol-5(9bH)-thion
8. 2-Ethyl-9b-(2,5-Dimethylphenyl)-2,3-dihydrooxazolo-[2,3-a]isoindol-5(9bH)-on
9. 8-Chlor-9b-(3-trifluormethylphenyl)-2,3-dihydrooxazolo-[2,3-a]isoindol-5(9bH)-on
10. 6-Methoxy-9b-(4-trifluormethylphenyl)-2,3-dihydrooxazolo-[2,3-a]isoindol-5(9bH)-on
11. 9b-(4-Hydroxyphenyl)-2,3-dihydrooxazolo-[2,3-a]isoindol-5(9bH)-thion
12. 8-Chlor-9b-(3-hydroxyphenyl)-2,3-dihydrooxazolo-[2,3-a]isoindol-5(9bH)-on
13. 7-Methylmercapto-9b-(4-ethoxyphenyl)-2,3-dihydrooxazolo-[2,3-a]isoindol-5(9bH)-on
14. 9-Methyl-9b-(3-methoxyphenyl)-2,3-dihydrooxazolo-[2,3-a]isoindol-5(9bH)-on
15. 8-Fluor-9b-(3-fluorphenyl)-2,3-dihydrooxazolo-[2,3-a]isoindol-5(9bH)-on
16. 9b-(4-Chlorphenyl)-2,3-dihydrooxazolo-[2,3-a]isoindol-5(9bH)-thion
17. 8-Methyl-9b-(3-methylsulfonylphenyl)-2,3-dihydrooxazolo-[2,3-a]isoindol-5(9bH)-on
18. 8-Chlor-9b-phenyl-2,3-dihydrooxazolo-[2,3-a]isoindol-5(9bH)-on-1-oxid
19. 8-Chlor-9b-benzyl-2,3-dihydrooxazolo-[2,3-a]isoindol-5(9bH)-on
20. 2,2,-Dimethyl-9b-phenethyl-2,3-dihydrooxazolo-[2,3-a]isoindol-5(9bH)-on
21. 9b-(3-Methylmercaptophenyl)-2,3-dihydrooxazolo-[2,3-a]isoindol-5(9bH)-on
22. 9b-(3-Methylaminophenyl)-2,3-dihydrooxazolo-[2,3-a]isoindol-5(9bH)-on
23. 9b-(3-Azidophenyl)-2,3-dihydrooxazolo-[2,3-a]isoindol-5(9bH)-on
24. 8-Methyl-9b-allyl-2,3-dihydrooxazolo-[2,3-a]isoindol-5(9bH)-on
25. 8-Chlor-9b-(3,5-dimethylphenyl)-2,3-dihydrooxazolo-[2,3-a]isoindol-5(9bH)-on
26. 8-Methyl-9b-(1-napthyl)-2,3-dihydrooxazolo-[2,3-a]isoindol-5(9bH)-on
27. 9b-(Anthracen-1-yl)-2,3-dihydrooxazolo-[2,3-a]isoindol-5(9bH)-thion
28. 9b-(Anthracen-9-yl)-2,3-dihydrooxazolo-[2,3-a]isoindol-5(9bH)-on
29. 9b-(Inden-1-yl)-2,3-dihydrooxazolo-[2,3-a]isoindol-5(9bH)-on
30. 9b-(Inden-3-yl)-2,3-dihydrooxazolo-[2,3-a]isoindol-5(9bH)-on
31. 9b-(Inden-4-yl)-2,3-dihydrooxazolo-[2,3-a]isoindol-5(9bH)-thion
32. 9b-(Phenanthren-1-yl)-2,3-dihydrooxazolo-[2,3-a]isoindol-5(9bH)-on
33. 9b-(Phenanthren-9-yl)-2,3-dihydrooxazolo-[2,3-a]isoindol-5(9bH)-on
34. 9b-(Cyclohexen-3-yl)-2,3-dihydrooxazolo-[2,3-a]isoindol-5(9bH)-thion
35. 9b-(2-Furyl)-2,3-dihydrooxazolo-[2,3-a]isoindol-5(9bH)-thion
36. 9b-(3-Furyl)-2,3-dihydrooxazolo-[2,3-a]isoindol-5(9bH)-on
37. 9b-(2-Thienyl)-2,3-dihydrooxazolo-[2,3-a]isoindol-5(9bH)-thion
38. 9b-(3-Thienyl)-2,3-dihydrooxazolo-[2,3-a]isoindol-5(9bH)-on
39. 9b-(Pyrimidin-4-yl)-2,3-dihydrooxazolo-[2,3-a]isoindol-5(9bH)-on
40. 9b-(Thiazol-2-yl)-2,3-dihydrooxazolo-[2,3-a]isoindol-5(9bH)-on
41. 9b-(Thiazol-4-yl)-2,3-dihydrooxazolo-[2,3-a]isoindol-5(9bH)-thion
42. 9b-(Indol-3-yl)-2,3-dihydrooxazolo-[2,3-a]isoindol-5(9bH)-on
43. 9b-(Indol-7-yl)-2,3-dihydrooxazolo-[2,3-a]isoindol-5(9bH)-on
44. 9b-(Chinolin-4-yl)-2,3-dihydrooxazolo-[2,3-a]isoindol-5(9bH)-on
45. 9b-(Chinolin-5-yl)-2,3-dihydrooxazolo-[2,3-a]isoindol-5(9bH)-thion
46. 9b-(Benzimidazol-4-yl)-2,3-dihydrooxazolo-[2,3-a]isoindol-5(9bH)-on
47. 9b-(Carbazol-1-yl)-2,3-dihydrooxazolo-[2,3-a]isoindol-5(9bH)-on
48. 9b-(Carbazol-4-yl)-2,3-dihydrooxazolo-[2,3-a]isoindol-5(9bH)-thion
49. 9b-(Phenothiazin-1-yl)-2,3-dihydrooxazolo-[2,3-a]isoindol-5(9bH)-thion
50. 9b-(Phenothiazin-4-yl)-2,3-dihydrooxazolo-[2,3-a]isoindol-5(9bH)-on
51. 9b-(4-Chinazolin-4-yl)-2,3-dihydrooxazolo-[2,3-a]isoindol-5(9bH)-on
52. 8-Chlor-9b-(inden-3-yl)-2,3-dihydrooxazolo-[2,3-a]isoindol-5(9bH)-on
53. 8-Methyl-9b-(isochinolin-1-yl)-2,3-dihydrooxazolo-[2,3-a]isoindol-5(9bH)-thion
54. 9-Methoxy-9b-(1-naphthyl)-2,3-dihydrooxazolo-[2,3-a]isoindol-5(9bH)-on
55. 9b-(Cumaron-3-yl)-2,3-dihydrooxazolo-[2,3-a]isoindol-5(9bH)-on

Verbindungen der Formel I, in der Y die Gruppe $-NR^7$ bedeutet, sind insbesondere die folgenden:

1. 8,9b-Dimethyl-2,3-dihydroimidazo-[2,1-a]isoindol-5(9bH)-on
2. 8-Chlor-9b-phenyl-2,3-dihydroimidazo-[2,1-a]isoindol-5(9bH)-on
3. 8-Fluor-9b-(4-methylpheny)-2,3-dihydroimidazo-[2,1-a]isoindol-5(9bH)-on
4. 8-Chlor-9b-(3-methylphenyl)-2,3-dihydroimidazo-[2,1-a]isoindol-5(9bH)-on
5. 3-Methyl-9b-(4-ethylphenyl)-2,3-dihydroimidazo-[2,1-a]isoindol-5(9bH)-on
6. 9b-(2,3-Dimethylphenyl)-2,3-dihydroimidazo-[2,1-a]isoindol-5(9bH)-thion
7. 8-Chlor-9b-(3,4-dimethylphenyl)-2,3-dihydroimidazo-[2,1-a]isoindol-5(9bH)-thion

8. 2-Ethyl-9b-(2,5-Dimethylphenyl)-2,3-dihydroimidazo-[2,1-a]isoindol-5(9bH)-on
9. 8-Chlor-9b-(3-trifluormethylphenyl)-2,3-dihydroimidazo-[2,1-a]isoindol-5(9bH)-on
10. 6-Methoxy-9b-(4-trifluormethylphenyl)-2,3-dihydroimidazo-[2,1 -a]isoindol-5(9bH)-on
11. 9b-(4-Hydroxyphenyl)-2,3-dihydroimidazo-[2,1-a]isoindol-5(9bH)-thion
12. 8-Chlor-9b-(3-hydroxphenyl)-2,3-dihydroimidazo-[2,1-a]isoindol-5(9bH)-on
13. 7-Methylmercapto-9b-(4-ethoxyphenyl)-2,3-dihydroimidazo-[2,1-a]isoindol-5(9bH)-on
14. 9-Methyl-9b-(3-methoxyphenyl)-2,3-dihydroimidazo-[2,1-a]isoindol-5(9bH)-on
15. 8-Fluor-9b-(3-fluorphenyl)-2,3-dihydroimidazo-[2,1-a]isoindol-5(9bH)-on
16. 9b-(4-Chlorphenyl)-2,3-dihydroimidazo-[2,1-a]isoindol-5(9bH)-thion
17. 8-Methyl-9b-(3-methylsulfonylphenyl)-2,3-dihydroimidazo-[2,1-a]isoindol-5(9bH)-on
18. 8-Chlor-9b-phenyl-2,3-dihydroimidazo-[2,1-a]isoindol-5(9bH)-on-1-oxid
19. 8-Chlor-9b-benzyl-2,3-dihydroimidazo-[2,1-a]isoindol-5(9bH)-on
20. 2,2,-Dimethyl-9b-phenethyl-2,3-dihydroimidazo-[2,1-a]isoindol-5(9bH)-on
21. 9b-(3-Methylmercaptophenyl)-2,3-dihydroimidazo-[2,1-a]isoindol-5(9bH)-on
22. 9b-(3-Methylaminophenyl)-2,3-dihydroimidazo-[2,1-a]isoindol-5(9bH)-on
23. 9b-(3-Azidophenyl)-2,3-dihydroimidazo-[2,1-a]isoindol-5(9bH)-on
24. 8-Methyl-9b-allyl-2,3-dihydroimidazo-[2,1-a]isoindol-5(9bH)-on
25. 8-Chlor-9b-(3,5-dimethylphenyl)-2,3-dihydroimidazo-[2,1-a]isoindol-5(9bH)-on
26. 8-Methyl-9b-(1-napthyl)-2,3-dihydroimidazo-[2,1-a]isoindol-5(9bH)-on
27. 9b-(Anthracen-1-yl)-2,3-dihydroimidazo-[2,1-a]isoindol-5(9bH)-thion
28. 9b-(Anthracen-9-yl)-2,3-dihydroimidazo-[2,1-a]isoindol-5(9bH)-on
29. 9b-(Inden-1-yl)-2,3-dihydroimidazo-[2,1-a]isoindol-5(9bH)-on
30. 9b-(Inden-3-yl)-2,3-dihydroimidazo-[2,1-a]isoindol-5(9bH)-on
31. 9b-(Inden-4-yl)-2,3-dihydroimidazo-[2,1-a]isoindol-5(9bH)-thion
32. 9b-(Phenanthren-1-yl)-2,3-dihydroimidazo-[2,1-a]isoindol-5(9bH)-on
33. 9b-(Phehanthren-9-yl)-2,3-dihydroimidazo-[2,1-a]isoindol-5(9bH)-on
34. 9b-(Cyclohexen-3-yl)-2,3-dihydroimidazo-[2,1-a]isoindol-5(9bH)-thion
35. 9b-(2-Furyl)-2,3-dihydroimidazo-[2,1-a]isoindol-5(9bH)-thion
36. 9b-(3-Furyl)-2,3-dihydroimidazo-[2,1-a]isoindol-5(9bH)-on
37. 9b-(2-Thienyl)-2,3-dihydroimidazo-[2,1-a]isoindol-5(9bH)-thion
38. 9b-(3-Thienyl)-2,3-dihydroimidazo-[2,1-a]isoindol-5(9bH)-on
39. 9b-(Pyrimidin-4-yl)-2,3-dihydroimidazo-[2,1-a]isoindol-5(9bH)-on
40. 9b-(Thiazol-2-yl)-2,3-dihydroimidazo-[2,1-a]isoindol-5(9bH)-on
41. 9b-(Thiazol-4-yl)-2,3-dihydroimidazo-[2,1-a]isoindol-5(9bH)-thion
42. 9b-(Indol-3-yl)-2,3-dihydroimidazo-[2,1-a]isoindol-5(9bH)-on
43. 9b-(Indol-7-yl)-2,3-dihydroimidazo-[2,1-a]isoindol-5(9bH)-on
44. 9b-(Chinolin-4-yl)-2,3-dihydroimidazo-[2,1-a]isoindol-5(9bH)-on
45. 9b-(Chinolin-5-yl)-2,3-dihydroimidazo-[2,1-a]isoindol-5(9bH)-thion
46. 9b-(Benzimidazol-4-yl)-2,3-dihydroimidazo-[2,1-a]isoindol-5(9bH)-on
47. 9b-(Carbazol-1-yl)-2,3-dihydroimidazo-[2,1-a]isoindol-5(9bH)-on
48. 9b-(Carbazol-4-yl)-2,3-dihydroimidazo-[2,1-a]isoindol-5(9bH)-thion
49. 9b-(Phenothiazin-1-yl)-2,3-dihydroimidazo-[2,1-a]isoindol-5(9bH)-thion
50. 9b-(Phenothiazin-4-yl)-2,3-dihydroimidazo-[2,1-a]isoindol-5(9bH)-on
51. 9b-(4-Chinazolin-4-yl)-2,3-dihydroimidazo-[2,1-a]isoindol-5(9bH)-on
52. 8-Chlor-9b-(inden-3-yl)-2,3-dihydroimidazo-[2,1-a]isoindol-5(9bH)-on
53. 8-Methyl-9b-(isochinolin-1-yl)-2,3-dihydroimidazo-[2,1-a]isoindol-5(9bH)-thion
54. 9-Methoxy-9b-(1-naphthyl)-2,3-dihydroimidazo-[2,1-a]isoindol-5(9bH)-on
55. 9b-(Cumaron-3-yl)-2,3-dihydroimidazo-[2,1-a]isoindol-5(9bH)-on

Beispiel 1

9b-(1-Naphthyl)-2,3-dihydrooxazolo-[2,3-a]isoindol-5(9bH)-on

2.76 g (10 mmol) 2-(1-Naphthoyl)benzoesäure wurden in 100 ml Xylol gelöst und nach Zugabe von 1.22 g (20 mmol) Ethanolamin sowie einer katalytischen Menge p-Toluolsulfonsäure 1 h am Wasserabscheider unter Rückfluß erhitzt. Dann wurde das Lösungsmittel im Vakuum entfernt und der Rückstand aus Ethanol umkristallisiert. Ausbeute 2.1 g (70 % d.Th.), Schmp. 144-146 °C.

Die verwendete 2-(1-Naphthoyl)benzoesäure wurde durch langsames Zutropfen von 1-Napthylmagnesiumbromid in Ether/Toluol 4/1 bei -10 °C zu einer Lösung von Phthalsäureanhydrid in Toluol, nach 2-stündigem Nachrühren Zugabe von ges. $NH_4Cl$-Lösung, Extraktion mit Essigester, Ausschütteln der Essigesterphase mit 2 N Sodalösung und erneuter Extraktion der angesäuerten Sodaphase mit Essigester hergestellt. Ausbeute nach Umkristallisation aus Ethanol 64 % d.Th., Schmp. 170 °C.

Analog zu Bsp. 1 wurden folgende Verbindungen hergestellt:

1.1 9b-(Anthracen-9-yl)-2,3-dihydrooxazolo-[2,3-a]isoindol-5(9bH)-on; Schmp. 205-206 ° C; Ausbeute 45 %. aus 2-(9-Anthracenoyl)bensoesäure und Ethanolamin

1.2 7,8-Dichlor-9b-(1-naphthyl)-2,3-dihydrooxazolo-[2,3-a]isoindol-5(9bH)-on; Schmp. 165-172 ° C; Ausbeute: 45 %. aus 4,5-Dichlor-2-benzoylbenzoesäure und Ethanolamin

1.3 9b-(2-Thienyl)-2,3-dihydrooxazolo[2,3-a]isoindol-5(9bH)-on; Schmp. 101-104 °C,
aus 2-(2-Thienoyl)bensoesäure und Ethanolamin (64 % Ausb.)

1.4 9b-(2-Furyl)-2,3-dihydrooxazolo[2,3-a]isoindol-5-(9bH)-on; aus 2-(2-Furoyl)benzoesäure und Ethanolamin

1.5 8-Methoxy-9b-phenyl-2,3-dihydrooxazolo-[2,3-a]isoindol-5-(9bH)-on;
aus 4-Methoxy-2 benzoylbenzoesäure und Ethanolamin

1.6 8-Chlor-9b-phenyl-2,3-dihydrooxazolo[2,3-a]isoindol-5(9bH)-on; Schmp. 112-114 °C, aus 4-Chlor-2-benzoylbenzoesäure und Ethanolamin (58 % Ausb.).

1.7 8-Methyl-9b-phenyl-2,3-dihydrooxazolo-[2,3-a]isoindol-5(9bH)-on; Schmp. 103-104 ° C; Ausbeute 60 %.
aus 4-Methyl-2-benzoylbenioesäure und Ethanolamin

1.8 8-Trifluormethyl-9b-phenyl-2,3-dihydrooxazolo-[2,3-a]isoindol-5-(9bH)-on;
aus 4-Trifluormethyl-2-benzoylbensoesäure und Ethanolamin

1.9 9b-(4-Pyridyl)-2,3-dihydrooxazolo[2,3-a]isoindol-5(9bH)-on; Schmp. 115-118 °C,
aus 2-(4-Pyridoyl)benzoesäure und Ethanolamin (62 % Ausb.)

1.10 9b-Methyl-2,3-dihydrooxazolo-[2,3-a]isoindol-5(9bH)-on; Öl; Ausbeute 61 %.
aus 2-Acetylbenzoesäure und Ethanolamin.

1.11 9b-Butyl-2,3-dihydrooxazolo-[2,3-a]isoindol-5(9bH)-on; Öl; Ausbeute 53 %.
aus 2-Butyrylbensoesäure und Ethanolamin

1.12 9b-Phenyl-2,3-dihydrooxazolo-[2,3-a]isoindol-5(9bH)-on; Schmp. 148-150 °C,
aus 2-Benioylbenzoesäure und Ethanolamin (75 % Ausb.).

1.13 9b-(4-Fluorphenyl)-2,3-dihydrooxazlo-[2,3-a]isoindol-5(9bH)-on; Schmp. 103-104 ° C; Ausbeute 64 %.
aus (4-Fluorbenzoyl)benzoesäure und Ethanolamin.

1.14 9b-(3-Methylphenyl)-2,3-dihydrooxazolo-[2,3-a]isoindol-5(9bH)-on; Schmp. 79-85 ° C; Ausbeute 45 %.
aus 2-(3-Methylbenioyl)benzoesäure und Ethanolamin.

1.15 9b-(3-Chlorphenyl)-2,3-dihydrooxazolo-[2,3-a]isoindol-5(9bH)-on; Schmp. 95-96 ° C; Ausbeute 72 %.
aus 2-(3-Chlorbenzoyl)benzoesäure und Ethanolamin.

1.16 9b-(3-Methoxyphenyl)-2,3-dihydrooxazolo-[2,3-a]isoindol-5(9bH)-on; Schmp. 120-121 ° C; Ausbeute 62 %.
aus 2-(3-Methoxybenzoyl)benzoesäure und Ethanolamin.

1.17 9b-(3-Trifluorphenyl)-2,3-dihydrooxazolo-[2,3-a]isoindol-5(9bH)-on; Schmp. 97-98 ° C; Ausbeute 46 %.
aus 2-(3-Trifluorbenzoyl)benzoesäure und Ethanolamin.

1.18 9b-(3,5-Dimethylphenyl)-2,3-dihydrooxazolo-[2,3-a]isoindol-5(9bH)-on;
aus 2-(3,5-Dimethylbenzoyl)benzoesäure und Ethanolamin.

1.19 9b-(3,5-Dichlorphenyl)-2,3-dihydrooxazolo-[2,3-a]isoindol-5(9bH)-on; Schmp. 158-159 ° C; Ausbeute 70 %.
aus 2-(3,5-Dichlorbenzoyl)benzoesäure und Ethanolamin.

1.20 9b-(4-Indanyl)-2,3-dihydrooxazolo-[2,3-a]isoindol-5(9bH)-on;Schmp. 153-157 ° C; Ausbeute 39 %.
aus 2-(4-Indanoyl)benzoesäure und Ethanolamin.

1.21 9b-(5-Tetralinyl)-2,3-dihydrooxazolo-[2,3-a]isoindol-5(9bH)-on;
aus 2-(5-Tetralinoyl)benzoesäure und Ethanolamin.

1.22 9b-(2-Benzothiophenyl)-2,3-dihydrooxazolo-[2,3-a]isoindol-5(9bH)-on;
aus 2-(2-Benzothiophenoyl)benzoesäure und Ethanolamin.

1.23 9b-(2-Benzofuranyl)-2,3-dihydrooxazolo-[2,3-a]isoindol-5(9bH)-on;
aus 2-(2-Benzofuranoyl)benzoesäure und Ethanolamin.

1.24 9b-(3-Indolyl)-2,3-dihydrooxazolo-[2,3-a)isoindol-5(9bH)-on;Schmp. 210-213° C; Ausbeute 39 %.
aus 2-(3-Indoloyl)benzoesäure und Ethanolamin.
1.25 9b-(4-Chinolinyl)-2,3-dihydrooxazolo-[2,3-a]isoindol-5(9bH)-on;
aus 2-(4-Chinolinoyl)benzoesäure und Ethanolamin.
1.26 9b-(1-Isochinolinyl)-2,3-dihydrooxazolo-[2,3-a]isoindol-5(9bH)-on;
aus 2-(1-Isochinolinoyl)bensoesäure und Ethanolamin.
1.27 9b-Phenyl-2,3-dihydrooxazolo[2,3-a]isoindol-5(9bH)-imin,; Schmp. 109-111° C; Ausbeute 47 %.
aus 2-Benzoyl-benzonitril und Ethanolamin.
1.28 9b-Phenyl-3-isopropyl-2,3-dihydrooxazolo[2,3-a]isoindol-5-(9bH)-on; Öl,
$[\alpha]_D^{20}$ = + 248,7 ($CHCl_3$)
aus 2-Benzoylbenzoesäure und S-(+)-Valinol (73 % Ausb.).
1.29 (+)-und(-)-9b-Phenyl-2-methyl-2,3-dihydrooxazolo[2,3-a]isoindol-5-(9bH)-on;
Smp. 147°C, $[\alpha]_D^{20}$ = + 137 ($CHCl_3$) bzw.
Smp. 154°C, $[\alpha]_D^{20}$ = - 263 ($CHCl_3$),
aus 2-Benzoylbenzoesäure und R-(-)-1-Amino-2-propanol nach Trennung an Cellulosetriacetat mit Methanol/Wasser 7:3.
1.30 (+)-und(-)-9b-Phenyl-2-methyl-2,3-dihydrooxazolo[2,3-a]isoindol-5-(9bH)-on;
Smp. 154°C, $[\alpha]_D^{20}$ = + 261,1 ($CHCl_3$) bzw.
Smp. 147°C, $[\alpha]_D^{20}$ = - 137 ($CHCl_3$),
aus 2-Benzoylbenzoesäure und S-(+)-1-Amino-2-propanol nach Trennung an RP 18 mit Methanol/Wasser 6:4.
1.31 9b-Phenyl-2,3-dimethyl-2,3-dihydrooxazolo[2,3-a]isoindol-5-(9bH)-on;
Smp. 76°C,
aus 2-Benzoylbenzoesäure und (+/-)-2-Amino-3-butanol
1.32 (+)-9b-Phenyl-3-methyl-2,3-dihydrooxazolo[2,3-a]isoindol-5-(9bH)-on;
Smp. 140-141°C, $[\alpha]_D^{20}$ = + 313.3 ($CHCl_3$),
aus 2-Benzoylbensoesäure und S-(+)-2-Amino-1-propanol
1.33 (-)-9b-Phenyl-3-methyl-2,3-dihydrooxazolo[2,3-a]isoindol-5-(9bH)-on;
Smp. 142-143°C, $[\alpha]_D^{20}$ = - 318,5 ($CHCl_3$),
aus 2-Benzoylbenzoesäure und R-(-)-2-Amino-1-propanol
1.34 9b-Phenyl-2,2-dimethyl-2,3-dihydrooxazolo[2,3-a]isoindol-5-(9bH)-on;
Smp. 149°C
aus 2-Benzoylbenzoesäure und 3-Amino-2-methyl-2-propanol (85 % Ausb.).
1.35 (+)-9b-Phenyl-3-methoxycarbonyl-2,3-dihydrooxazolo[2,3-a]isoindol-5-(9bH)-on;
Smp.
aus 2-Benzoylbenzoesäure und L-Serinmethylester
1.36 9b-Phenyl-2,3-dihydrooxazolo[2,3-a]isoindol-5(9bH)-imin,;
aus 2-Benzoyl-benzonitril und Ethanolamin.

Beispiel 2

9b-Phenyl-2,3-dihydrooxazolo-[2,3-a]isoindol-5(9bH)-thion

1.9 g (7.5 mmol) 9b-Phenyl-2,3-dihydrooxazolo-[2,3-a]isoindolin-5(9bH)-on (Bsp. 1.12) in 100 ml abs. Dioxan wurden mit 3.8 g (9.4 mmol) Lawesson's-Reagenz [2,4-Bis-(4-methoxyphenyl)-1,3-dithia-2,4-diphosphetan-2,4-disulfid] versetzt und 5 h bei 60°C gerührt (DC-Kontrolle).

Nach dem Abkühlen wurde von Niederschlag abfiltriert, das Filtrat im Vakuum eingedampft und der Rückstand durch Flash-Säulenchromatographiemit Heptan/Methylethylketon 6/1 als Eluens gereinigt.

Beispiel 3

Enantiomerentrennung von rac-8-Chlor-9b-phenyl-2,3-dihydrooxazolo-[2,3-a]isoindol-5(9bH)-on (Bsp. 1.6) an Cellulosetriacetat

Für die Trennung der Antipoden wurden 200 mg des Racemats in 15 ml Ethanol gelöst, auf eine Säule mit 50 mm Innendurchmesser und 300 mm Länge (entsprechend 250 g Cellulosetriacetat, 15-25 Korngröße, Merck 16326) aufgegeben und mit Ethanol eluiert (Fluß 7.5 ml/min, ca. 1.5 bar).

| | Peak I | Peak II |
|---|---|---|
| UV-Detektion [nm]: | 254 | 254 |
| $[\alpha]_D^{20}$ * : | + 114.8 | - 115.2 |
| Schmp. [°C] : | 89-91 | 89-91 |

Die Enantiomeren wurden aus Ethanol umkristallisiert.

* Enantiomerenreinheit nach HPLC jeweils >99.6 % ee

Beispiel 4

9b-Phenyl-2,3-dihydroimidazo-[2,1-a]isoindol-5(9bH)-on

5.0 g (22 mmol) 2-Benzoylbenzoesäure wurden in 100 ml Toluol gelöst und nach Zugabe von 6.6 g (110 mmol) Ethylendiamin sowie einer katalytischen Menge p-Toluolsulfonsäure 12 h am Wasserabscheider unter Rückfluß erhitzt. Dann wurde das Lösungsmittel im Vakuum entfernt und der Rückstand aus Ethanol umkristallisiert. Ausbeute 3.5 g (63 % d.Th.), Schmp. 152-154 °C.

Beispiel 5

1-Acetyl-9b-phenyl-2,3-dihydroimidazo-[2,1-a]isoindol-5(9bH)-on

1 g (4 mmol) der in Beispiel 4 erhaltenen Verbindung wurden mit 10 ml Essigsäureanhydrid 8 h bei Raumtemperatur gerührt. Man gießt auf Wasser, saugt den ausgefallenen Rückstand ab und wäscht die Kristalle mit Ether. Ausbeute: 1.1 g (92 % d.Th.), Schmp. 171-173 °C.

Beispiel 6

1-Methyl-9b-phenyl-2,3-dihydroimidazo-[2,1-a]isoindol-5(9bH)-on

1 g (4 mmol) der in Beispiel 4 erhaltenen Verbindung wurde in 5 ml DMF gelöst und mit 0.5 ml Methyljodid und 0.13 g NaH (100proz.) versetzt. Nach vierstündigem Rühren wurde nochmals 0.5 ml Methyljodid und 0.13 g NaH (100proz.) zugesetzt. Nach weiteren 2 h wurde die Reaktionslösung auf Wasser gegeben, mit Essigester extrahiert, getrocknet und das Lösungsmittel im Vakuum eingedampft. Nach Säulenchromatographie an Kieselgel (Laufmittel:Essigester/Isohexan, 1:2) konzipiert man die gewünschten Fraktionen und kristallisiert den Rückstand aus Isohexan und einigen Tropfen Ethanol. Ausbeute:0.59 g (56 % d.Th.), Schmp. 119-121 °C.

Beispiel 7

Hemmung der HIV Reversen Transkriptase (RT) durch Derivate des 9b-Phenyl-2,3-dihydrooxazolo-[2,3-a]-isoindol-5(9bH)-on und das 9b-Phenyl-2 3-dihydro-imidazo-[2,1-a]isoindol-5(9bH)-on.

Das Screeningtestsystem beinhaltet die gereinigte RT aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [$^3$H]-Thymidin-5'-triphosphat-Einbau durch Auszählen im $\beta$-Counter. In der folgenden Tabelle wird für die untersuchten

Verbindungen der IC50-Wert angegeben. Dieser Wert entspricht derjenigen Konzentration der Testsubstanz, die eine Hemmung der Reversen Transkriptase Aktivität um 50% bewirkt.

Ergebnisse:

| Substanz | Hemmung der HIV-RT $IC_{50}$[M] |
|---|---|
| 9b-Phenyl-2,3-dihydrooxazolo-[2,3-a]isoindol-5(9bH)-on | $6.1 \times 10^{-6}$ |
| 7,8-Dichlor-9b-phenyl-2,3-dihydrooxazolo-[2,3,-a]isoindol-5(9bH)-on | $14.1 \times 10^{-6}$ |
| 9b-(1-Naphthyl)-2,3-dihydrooxazolo-[2,3,-a]isoindol-5(9bH)-on | $1.8 \times 10^{-6}$ |
| 9b-(3-Methylphenyl)-2,3-dihydrooxazolo-[2,3,-a]isoindol-5(9bH)-on | $7.9 \times 10^{-6}$ |

| Substanz | Hemmung der HIV-RT $IC_{50}$[M] |
|---|---|
| 8-Chlor-9b-phenyl-2,3-dihydrooxazolo-[2,3-a]-isoindol-5(9bH)-on | $5.7 \times 10^{-6}$ |
| 9b-(3-Chlorphenyl)-2,3-dihydroxazolo-[2,3-a]isoindol-5(9bH)-on | $2.1 \times 10^{-6}$ |
| 9b-(3,5-Dichlorphenyl)-2,3-dihydrooxazolo-[2,3-a]isoindol-5(9bH)-on | $2.2 \times 10^{-6}$ |
| 9b-(3-Indolyl)-2,3-dihydrooxazolo-[2,3-a]isoindol-5(9bH)-on | $7.3 \times 10^{-6}$ |

**Patentansprüche**

**1.** Verwendung von Oxazolo[2,3-a]isoindol- und Imidazo[2,1-a]isoindol-Derivate der allgemeinen Formel I

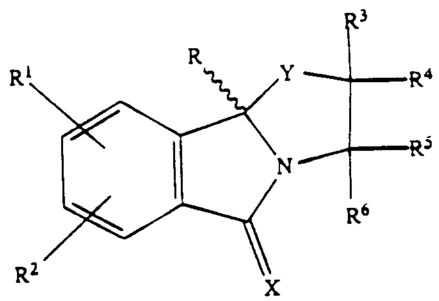

(I),

zur Herstellung von Arzneimitteln mit antiviraler Wirkung, wobei

X ein Sauerstoff- oder Schwefelatom, die Iminogruppe =NH oder eine =N-$C_1$-$C_5$-Alkyliminogruppe sein kann,

Y ein Sauerstoffatom oder die Gruppe $NR^7$ sein kann, wobei $R^7$ ein Wasserstoffatom oder einen $C_1$-$C_6$-Alkyl oder $C_1$-$C_6$-Acylrest bedeutet,

R ein Wasserstoffatom, einen geradkettigen oder verzweigten, gesättigten oder ungesättigten aliphatischen Rest mit 1-9 C-Atomen, der durch Phenyl substituiert sein kann, oder einen $C_1$-$C_6$-Alkoxy-$C_1$-$C_6$-alkyl- oder $C_1$-$C_6$-Alkylmercapto-$C_1$-$C_6$-alkylrest bedeutet, oder
einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsufinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylamino-carbonyl, $C_1$-$C_6$-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl, oder
einen mono-, bi- oder tricyclischen carbocyclischen Ring mit 7-15 C-Atomen oder ein

heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind,

$R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Trifluormethyl, Carboxy, Halogen, Hydroxy, Nitro, Cyano, Azido, Phenyl oder Benzyloxy bedeutet,

$R^2$ die gleiche Bedeutung hat wie $R^1$, wobei die Reste $R^1$ und $R^2$ unabhängig voneinander gleich oder verschieden sein können,

$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Halogen, Cyano, Hydroxy, Carboxy, $C_1$-$C_6$-Alkoxycarbonyl,Aminocarbonyl, $C_1$-$C_6$-Alkylaminocarbonyl oder Di-$C_1$-$C_6$-alkylaminocarbonyl bedeutet,

$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,

sowie deren Tautomere, Enantiomere, Diastereomere und physiologisch verträgliche Salze.

**2.** Verwendung gemäß Anspruch 1, dadurch gekennzeichnet, daß R einen carbocyclischen Ring mit 7-15 C-Atomen bedeutet, ausgewählt aus der Gruppe Naphthyl-, Anthracenyl-, Phenanthrenyl-, Flourenyl-, Indenyl-, Indanyl-, Acenaphthylenyl-, Norbornyl-, Adamantylring oder eine $C_3$-$C_7$-Cycloalkyl oder $C_5$-$C_8$-Cycloalkenylgruppe, wobei diese partiell hydriert oder vollständig hydriert sein können.

**3.** Verwendung gemäß Anspruch 1, dadurch gekennzeichnet, daß R einen heterocylischen mono-, bi- oder tricyclischen Ring bedeutet, ausgewählt aus der Gruppe Pyridinyl-, Pyrimidinyl, Pyridazinyl-, Pyrazinyl-, Triazinyl-, Pyrrolyl-, Pyrazolyl-, Imidazolyl-, Triazolyl-, Thiazolyl-, Oxazolyl-, Isoxazolyl-, Oxadiazolyl-, Furazanyl-, Furanyl-, Thiophenyl-, Indolyl-, Chinolinyl-, Isochinolinyl-, Cumaronyl-, Thionaphthenyl-, Benzoxazolyl-, Benzthiazolyl-, Indazolyl-, Benzimidazolyl-, Benztriazolyl-, Chromenyl-, Phthalazinyl, Chinazolinyl-, Chinoxalinyl-, Methylendioxy-benzolyl-, Carbazolyl-, Acridinyl-, Phenoxazinyl-, Phenothiazinyl-, Phenazinyl- oder Puringruppe, wobei die Heterocyclen partiell oder vollständig hydriert sein können.

**4.** Verwendung gemäß Anspruch 1, dadurch gekennzeichnet, daß

X ein Sauerstoff- oder Schwefelatom bedeutet, und

Y ein Sauerstoffatom oder $-NR^7$ bedeutet, wobei $R^7$ Wasserstoff oder $C_1$-$C_6$-Alkyl- oder $C_1$-$C_6$-Acylrest sein kann, und

R unsubstituiertes Phenyl oder Phenyl ein- oder zweifach substituiert durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_1$-$C_6$-Alkenyl, $C_2$-$C_6$-Alkinyl, $C_3$-$C_6$-Alkenyl-oxy, $C_1$-$C_6$-Alkylamino, $C_1$-$C_6$-Dialkylamino-, $C_1$-$C_6$-Alkylcarbonylamino, $C_1$-$C_6$-Alkylaminocarbonyl, $C_1$-$C_6$-Alkoxycarbonyl, Amino, Hydroxy, Nitro, Azido, Trifluormethyl, Cyano oder Halogen bedeutet, oder

Biphenyl, Naphthyl, Anthracenyl, Indenyl, Fluorenyl, Acenaphthylenyl, Phenanthrenyl, Norbornyl, Adamantyl, $C_3$-$C_6$-Cycloalkyl, $C_5$-$C_8$-Cycloalkenyl bedeutet, oder

Pyrrolyl, Imidazolyl, Furanyl, Thiophenyl, Pyridinyl, Pyrimidinyl, Thiazolyl, Triazinyl, Indolyl, Chinolinyl, Isochinolinyl, Cumaronyl, Thionaphthenyl, Benzimidazolyl, Chinazolinyl, Methylendioxybenzolyl, Ethylendioxybenzolyl, Carbazolyl, Acridinyl oder Phenothiazinyl bedeutet, und

$R^1$ und $R^2$ Wasserstoff, $C_1$-$C_6$-Alkyl, $C_2$-$C_6$-Alkenyl, $C_2$-$C_6$-Alkinyl, $C_1$-$C_6$-Alkoxy, $C_1$-$C_6$-Alkylmercapto, $C_1$-$C_6$-Alkylamino, $C_1$-$C_6$-Alkoxycarbonyl, Trifluormethyl, Amino, Halogen, Hydroxy, Cyano und Azido bedeutet,

$R^3$, $R^4$, $R^5$ und $R^6$ Wasserstoff, $C_1$-$C_6$-Alkyl, $C_1$-$C_6$-Alkoxy, $C_1$-$C_6$-Alkylmercapto, Carboxy, $C_1$-$C_6$-Alkoxycarbonyl, Halogen, Cyano und Hydroxy bedeuten.

**5.** Oxazolo[2,3-a]-isoindol-Derivate der allgemeinen Formel Iα

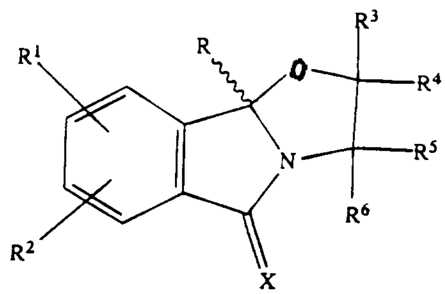

(Ia),

in der

X ein Sauerstoff- oder Schwefelatom, die Iminogruppe =NH oder eine $=N-C_1-C_5$-Alkyliminogruppe sein kann,

R ein Wasserstoffatom, einen geradkettigen oder verzweigten, gesättigten oder ungesättigten aliphatischen Rest mit 1-9 C-Atomen, der durch Phenyl substituiert sein kann, oder einen $C_1$-$C_6$-Alkoxy-$C_1$-$C_6$-alkyl- oder $C_1$-$C_6$-Alkylmercapto-$C_1$-$C_6$-alkylrest bedeutet, oder
einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylamino-carbonyl, $C_1$-$C_6$-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl, oder
einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind,

$R^1$ einen geradkettigen oder verzweigten ungesättigten aliphatischen Rest mit bis zu 6 C-Atomen, $C_1$-$C_6$-Alkylmercapto, $C_1$-$C_6$-Alkylsulfinyl, $C_1$-$C_6$-Alkylsulfonyl, $C_1$-$C_6$-Alkylamino, Di-$C_1$-$C_6$-Alkylamino, Sulfonamido, $C_1$-$C_6$-Alkoxycarbonyl, Carboxy, Hydroxy, Cyano, Azido, Phenyl oder Benzyloxy bedeutet,

$R^2$ ein Wasserstoffatom bedeutet oder die gleiche Bedeutung hat wie $R^1$,

$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-C1-C6-Alkylamino, Halogen, Cyano, Hydroxy, Carboxy, $C_1$-$C_6$-Alkoxycarbonyl,Aminocarbonyl, $C_1$-$C_6$-Alkylaminocarbonyl oder ni-$C_1$-$C_6$-alkylaminocarbonyl bedeutet,

$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,

sowie deren Tautomere, Enantiomere, Diastereomere und physiologisch verträgliche Salze.

**6.** R- und S-Oxazolo[2,3-a]isoindol- und R- und S- Imidazo[2,1-a]isoindol-Derivate der allgemeinen Formel I

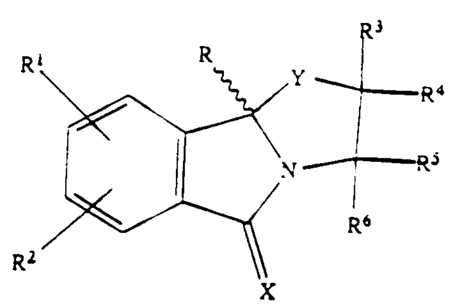

(I),

in der

X ein Sauerstoff- oder Schwefelatom, die Iminogruppe =NH oder eine $=N-C_1-C_5$-alkylimiogruppe sein kann,

Y ein Sauerstoffatom oder die Gruppe $NR^7$ sein kann, wobei $R^7$ ein Wasserstoffatom oder einen $C_1$-$C_6$-Alkyl oder $C_1$-$C_6$-Acylrest bedeutet,

R ein Wasserstoffatom, einen geradkettigen oder verzweigten, gesättigten oder ungesättigten aliphatischen Rest mit 1-9 C-Atomen, der durch Phenyl substituiert sein kann, oder einen $C_1$-$C_6$-Alkoxy-$C_1$-$C_6$-alkyl- oder $C_1$-$C_6$-Alkylmercapto-$C_1$-$C_6$-alkylrest bedeutet, oder

einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylamino-carbonyl, $C_1$-$C_6$-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl, oder

einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind,

$R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Trifluormethyl, Carboxy, Halogen, Hydroxy, Nitro, Cyano, Azido, Phenyl oder Benzyloxy bedeutet,

$R^2$ die gleiche Bedeutung hat wie $R^1$, wobei die Reste $R^1$ und $R^2$ unabhängig voneinander gleich oder verschieden sein können,

$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Halogen, Cyano, Hydroxy, Carboxy, $C_1$-$C_6$-Alkoxycarbonyl,Aminocarbonyl, $C_1$-$C_6$-Alkylaminocarbonyl oder Di-$C_1$-$C_6$-alkylaminocarbonyl bedeutet,

$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,

sowie deren Tautomere, Diastereomere und physiologisch verträgliche Salze.

**7.** Arzneimittel enthaltend mindestens eine Verbindung der Formel I oder Ia gemäß Anspruch 5 oder 6 neben pharmakologisch verträglichen Hilfs- oder Trägerstoffen.

**8.** Verwendung von Verbindungen der Formel I oder Ia gemäß Anspruch 5 oder 6 zur Herstellung von Arzneimitteln zur Behandlung von viralen oder retroviralen Infektionen oder von durch diese Infektionen verursachten Erkrankungen wie AIDS und ARC.

**9.** Verfahren zur Herstellung von Arzneimitteln enthaltend mindestens eine Verbindung der Formel I oder Ia gemäß Anspruch 5 oder 6 neben pharmazeutisch üblichen Träger- oder Hilfsstoffen, dadurch gekennzeichnet, daß man eine Verbindung der Formel I oder Ia mit den Träger- oder Hilfsstoffen vermischt und zu entsprechenden Darreichungsformen verarbeitet.

## Claims

**1.** Use of oxazolo-[2,3-a]-isoindole and imidazo-[2,1-a]-isoindole derivatives of the general formula I

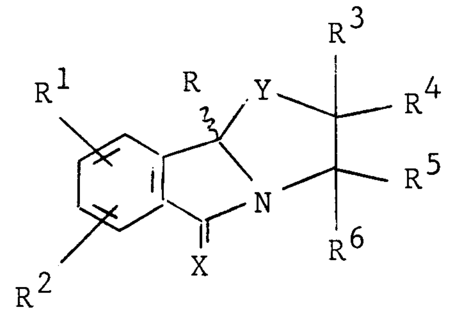

(I),

for the preparation of medicaments with antiviral action, whereby X can be an oxygen or sulphur atom, the imino group =NH or an =N-$C_1$-$C_5$-alkylimino group, Y can be an oxygen atom or the group $NR^7$, whereby $R^7$ signifies a hydrogen atom or a $C_1$-$C_6$-alkyl or $C_1$-$C_6$-acyl radical, R signifies a hydrogen atom, a straight-chained or branched, saturated or unsaturated aliphatic radical with 1-9 C-atoms, which can be substituted by phenyl, or a $C_1$-$C_6$-alkoxy-$C_1$-$C_6$-alkyl or $C_1$-$C_6$-alkylmercapto-$C_1$-$C_6$-alkyl radical or signifies a phenyl ring which is possibly substituted one or more times by $C_1$-$C_6$-alkyl, $C_1$-$C_6$-alkoxy, $C_1$-$C_6$-alkylmercapto, $C_1$-$C_6$-alkylsulphinyl, $C_1$-$C_6$-alkylsulphonyl, $C_2$-$C_6$-alkenyl, $C_2$-$C_6$-alkynyl, $C_2$-$C_6$-alkenyloxy, $C_2$-$C_6$-alkenylmercapto, $C_2$-$C_6$-alkynyloxy, $C_2$-$C_6$-alkynylmercapto, amino, $C_1$-$C_6$-alkylamino, di-$C_1$-$C_6$-alkylamino, $C_1$-$C_6$-alkylcarbonylamino, $C_1$-$C_6$-alkylaminocarbonyl, $C_1$-$C_6$-alkoxycarbonyl, hydroxyl, benzyloxy, phenylmercapto, phenyloxy, nitro, cyano, halogen, trifluoromethyl, azido, formylamino, carboxyl or phenyl, or a mono-, bi- or tricyclic carbocyclic ring with 7 - 15 C-atoms or a heterocyclic mono-, bi- or tricyclic ring system with, in each case, 5 or 6 ring atoms and, per ring system, can contain 1 - 4 or 1 - 5 heteroatoms, respectively, whereby the heteroatoms are nitrogen, sulphur or oxygen, $R^1$ signifies a hydrogen atom, a straight-chained or branched, saturated or unsaturated aliphatic radical with 1 - 6 C-atoms or $C_1$-$C_6$-alkoxy,$C_1$-$C_6$-alkylmercapto, $C_1$-$C_6$-alkylsulphinyl, $C_1$-$C_6$-alkylsulphonyl, amino, $C_1$-$C_6$-alkylamino, di-$C_1$-$C_6$-alkylamino, sulphonamido, $C_1$-$C_6$-alkoxycarbonyl, trifluoromethyl, carboxyl, halogen, hydroxyl, nitro, cyano, azido, phenyl or benzyloxy, $R^2$ has the same meaning as $R^1$, whereby the radicals $R^1$ and $R^2$, independently of one nother, can be the same or different, $R^3$ signifies hydrogen, $C_1$-$C_6$-alkyl, $C_1$-$C_6$-alkoxy, $C_1$-$C_6$-alkylmercapto, amino, $C_1$-$C_6$-alkylamino, di-$C_1$-$C_6$-alkylamino, halogen, cyano, hydroxyl, carboxyl, $C_1$-$C_6$-alkoxycarbonyl, aminocarbonyl, $C_1$-$C_6$-alkylaminocarbonyl or di-$C_1$-$C_6$-alkylaminocarbonyl, $R^4$, $R^5$, $R^6$ have the same meaning as $R^3$, whereby the radicals $R^3$, $R^4$, $R^5$ and $R^6$, independently of one another, can be the same or different, as well as their tauromers, enantiomers, diastereomers and physiologically compatible salts.

**2.** Use according to claim 1, characterised in that R signifies a carbocyclic ring with 7 - 15 C-atoms selected from the group naphthyl, anthracenyl, phenanthrenyl, fluorenyl, indenyl, indanyl, acenaphthylenyl, norbornyl, adamantyl ring or a $C_3$-$C_7$-cycloalkyl or $C_5$-$C_8$-cycloalkenyl group, whereby these can be partly hydrogenated or fully hydrogenated.

**3.** Use according to claim 1, characterised in that R signifies a heterocyclic mono-, bi- or tricyclic ring selected from the group pyridinyl, pyrimidinyl, pyridazinyl, pyrazinyl, triazinyl, pyrrolyl, pyrazolyl, imidazolyl, triazolyl, thiazolyl, oxazolyl, isoxazolyl, oxadiazolyl, furazanyl, furanyl, thiophenyl, indolyl, quinolinyl, isoquinolinyl, cumaronyl, thionaphthenyl, benzoxazolyl, benzthiazolyl, indazolyl, benzimidazolyl, benztriazolyl, chromenyl, phthalazinyl, quinazolinyl, quinoxalinyl, methylenedioxybenzolyl, carbazolyl, acridinyl, phenoxazinyl, phenothiazinyl, phenazinyl or purine group, whereby the hiterocycles can be partly or completely hydrogenated.

**4.** Use according to claim 1, characterised in that X signifies an oxygen or sulphur atom and Y signifies an oxygen atom or -$NR^7$, whereby $R^7$ can be hydrogen or $C_1$-$C_6$-alkyl or $C_1$-$C_6$-acyl radical and R signifies unsubstituted phenyl or phenyl substituted once or twice by $C_1$-$C_6$-alkyl, $C_1$-$C_6$-alkoxy, $C_1$-$C_6$-alkylmercapto, $C_1$-$C_6$-alkylsulphinyl, $C_1$-$C_6$-alkylsulphonyl, $C_2$-$C_6$-alkenyl, $C_2$-$C_6$-alkynyl, $C_3$-$C_6$-al-

kenyloxy, $C_1$-$C_6$-alkylamino, $C_1$-$C_6$-dialkylamino, $C_1$-$C_6$-alkylcarbonylamino, $C_1$-$C_6$-alkylaminocarbonyl, $C_1$-$C_6$-alkoxycarbonyl, amino, hydroxyl, nitro, azido, trifluoromethyl, cyano or halogen, or signifies biphenyl, naphthyl, anthracenyl, indenyl, fluorenyl, acenaphthylenyl, phenanthrenyl, norbornyl, adamantyl, $C_3$-$C_6$-cycloalkyl, $C_5$-$C_8$-cycloalkenyl, or signifies pyrrolyl, imidazolyl, furanyl, thiophenyl, pyridinyl, pyrimidinyl, thiazolyl, triazinyl, indolyl, quinolinyl, isoquinolinyl, cumaronyl, thionaphthenyl, benzimidazolyl, quinazolinyl, methylenedioxybenzolyl, ethylenedioxybenzolyl, carbazolyl, acridinyl or phenothiazinyl, and $R^1$ and $R^2$ signify hydrogen, $C_1$-$C_6$-alkyl, $C_2$-$C_6$-alkenyl, $C_2$-$C_6$-alkynyl, $C_1$-$C_6$-alkoxy, $C_1$-$C_6$-alkylmercapto, $C_1$-$C_6$-alkylamino, $C_1$-$C_6$-alkoxycarbonyl, trifluoromethyl, amino, halogen, hydroxyl, cyano and azido, $R^3$, $R^4$, $R^5$ and $R^6$ signify hydrogen, $C_1$-$C_6$- alkyl, $C_1$-$C_6$-alkoxy, $C_1$-$C_6$-alkylmercapto, carboxyl, $C_1$-$C_6$-alkoxycarbonyl, halogen, cyano and hydroxyl.

**5.** Oxazolo-[2,3-a]-isoindole derivatives of the general formula Ia

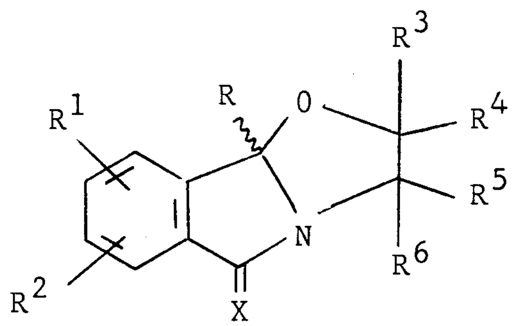

(Ia)

in which X can be an oxygen or sulphur atom, the imino group =NH or an =N-$C_1$-$C_5$-alkylimino group, R signifies a hydrogen atom, a straight-chained or branched, saturated or unsaturated aliphatic radical with 1 - 9 C-atoms, which can be substituted by phenyl, or a $C_1$-$C_6$-alkoxy-$C_1$-$C_6$-alkyl or $C_1$-$C_6$-alkylmercapto-$C_1$-$C_6$-alkyl radical, or signifies a phenyl ring which is possibly substituted one or more times by $C_1$-$C_6$-alkyl, $C_1$-$C_6$-alkoxy, $C_1$-$C_6$-alkylmercapto, $C_1$-$C_6$-alkylsulphinyl, $C_1$-$C_6$-alkylsulphonyl, $C_2$-$C_6$-alkenyl, $C_2$-$C_6$-alkynyl, $C_2$-$C_6$-alkenyloxy, $C_2$-$C_6$-alkenylmercapto, $C_2$-$C_6$-alkynyloxy, $C_2$-$C_6$-alkynylmercapto, amino, $C_1$-$C_6$-alkylamino, di-$C_1$-$C_6$-alkylamino, $C_1$-$C_6$ alkylcarbonylamino, $C_1$-$C_6$-alkylaminocarbonyl, $C_1$-$C_6$-alkoxycarbonyl, hydroxyl, benzyloxy, phenylmercapto, phenyloxy, nitro, cyano, halogen, trifluoromethyl, azido, formylamino, carboxyl or phenyl, or signifies a mono-, bi- or tricyclic carbocyclic ring with 7 - 15 C-atoms or a heterocyclic mono-, bi- or tricyclic ring system with, in each case, 5 or 6 ring atoms and, per ring system, can contain 1 - 4 or 1 - 5 heteroatoms, respectively, whereby the heteroatoms are nitrogen, sulphur or oxygen, $R^1$ signifies a straight-chained or branched unsaturated aliphatic radical with up to 6 C-atoms, $C_1$-$C_6$-alkylmercapto, $C_1$-$C_6$-alkylsulphinyl, $C_1$-$C_6$-alkylsulphonyl, $C_1$-$C_6$-alkylamino, di-$C_1$-$C_6$-alkylamino, sulphonamido, $C_1$-$C_6$-alkoxycarbonyl, carboxyl, hydroxyl, cyano, azido, phenyl or benzyloxy, $R^2$ signifies a hydrogen atom or has the same meaning as $R^1$, $R^3$ signifies hydrogen, $C_1$-$C_6$-alkyl, $C_1$-$C_6$-alkoxy, $C_1$-$C_6$-alkylmercapto, amino, $C_1$-$C_6$-alkylamino, di-$C_1$-$C_6$-alkylamino, halogen, cyano, hydroxyl, carboxyl, $C_1$-$C_6$-alkoxycarbonyl, aminocarbonyl, $C_1$-$C_6$-alkylaminocarbonyl or di-$C_1$-$C_6$-alkylaminocarbonyl, $R^4$, $R^5$, $R^6$ have the same meaning as $R^3$, whereby the radicals $R^3$, $R^4$, $R^5$ and $R^6$, independently of one another, can be the same or different, as well as their tautomers, enantiomers, diastereomers and physiologically compatible salts.

**6.** R- and S-oxazolo-[2,3-a]-isoindole and R- and S-imidazo-[2,1-a]-isoindole derivatives of the general formula I

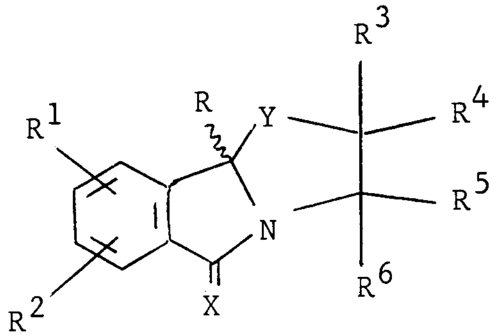

(I)

in which X can be an oxygen or sulphur atom, the imino group =NH or an =N-$C_1$-$C_5$-alkylimino group, Y can be an oxygen atom or the group $NR^7$, whereby $R^7$ signifies a hydrogen atom or a $C_1$-$C_6$-alkyl or $C_1$-$C_6$-acyl radical, R signifies a hydrogen atom, a straight-chained or branched, saturated or unsaturated aliphatic radical with 1 - 9 C-atoms, which can be substituted by phenyl, or a $C_1$-$C_6$-alkoxy-$C_1$-$C_6$-alkyl or $C_1$-$C_6$-alkylmercapto-$C_1$-$C_6$-alkyl radical or signifies a phenyl ring which is possibly substituted one or more times by $C_1$-$C_6$-alkyl, $C_1$-$C_6$-alkoxy, $C_1$-$C_6$-alkylmercapto, $C_1$-$C_6$-alkylsulphinyl, $C_1$-$C_6$-alkylsulphonyl, $C_2$-$C_6$-alkenyl, $C_2$-$C_6$-alkynyl, $C_2$-$C_6$-alkenyloxy, $C_2$- $C_6$-alkenylmercapto, $C_2$-$C_6$-alkynyloxy, $C_2$-$C_6$-alkynylmercapto, amino, $C_1$-$C_6$-alkylamino, di-$C_1$-$C_6$-alkylamino, $C_1$-$C_6$-alkylcarbonylamino, $C_1$-$C_6$-alkylaminocarbonyl, $C_1$-$C_6$-alkoxycarbonyl, hydroxyl, benzyloxy, phenylmercapto, phenyloxy, nitro, cyano, halogen, trifluoromethyl, azido, formylamino, carboxyl or phenyl, or a mono-, bi- or tricyclic carbocyclic ring with 7 - 15 C-atoms or a heterocyclic mono-, bi- or tricyclic ring system with, in each case, 5 or 6 ring atoms and, per ring system, can contain 1 - 4 or 1 - 5 heteroatoms, respectively, whereby the heteroatoms are nitrogen, sulphur or oxygen, $R^1$ signifies a hydrogen atom, a straight-chained or branched, saturated or unsaturated aliphatic radical with 1 - 6 C-atoms or $C_1$ -$C_6$-alkoxy, $C_1$-$C_6$-alkylmercapto, $C_1$-$C_6$-alkylsulphinyl, $C_1$-$C_6$-alkylsulphonyl, amino, $C_1$-$C_6$-alkylamino, di-$C_1$-$C_6$-alkylamino, sulphonamido, $C_1$-$C_6$-alkoxycarbonyl, trifluoromethyl, carboxyl, halogen, hydroxyl, nitro, cyano, azido, phenyl or benzyloxy, $R^2$ has the same meaning as $R^1$, whereby the radicals $R^1$ and $R^2$, independently of one another, can be the same or different, $R^3$ signifies hydrogen, $C_1$-$C_6$-alkyl, $C_1$-$C_6$-alkoxy, $C_1$-$C_6$-alkylmercapto, amino, $C_1$-$C_6$-alkylamino, di-$C_1$-$C_6$-alkylamino, halogen, cyano, hydroxyl, carboxyl, $C_1$-$C_6$-alkoxycarbonyl, aminocarbonyl, $C_1$-$C_6$-alkylaminocarbonyl or di-$C_1$-$C_6$-alkylaminocarbonyl, $R^4$, $R^5$, $R^6$ have the same meaning as $R^3$, $R^4$, $R^5$ and $R^6$, independently of one another, can be the same or different, as well as their tautomers, diastereomers and physiologically compatible salts.

**7.** Medicaments containing at least one compound of the formula I or Ia according to claim 5 or 6, besides pharmacologically compatible adjuvant and carrier materials.

**8.** Use of compounds of the formula I or Ia according to claim 5 or 6 for the preparation of medicaments for the treatment of viral or retroviral infections or of diseases caused by these infections, such as AIDS and ARC.

**9.** Process for the production of medicaments containing at least one compound of the formula I or Ia according to claim 5 or 6, besides pharmaceutically usual carrier and adjuvant materials, characterised in that one mixes a compound of the formula I or Ia with the carrier or adjuvant materials and works up to appropriate forms of administration.

## Revendications

1. Utilisation de dérivés d'oxazolo[2,3-a]isoindol et d'imidazo[2,1-a]isoindol de formule générale I

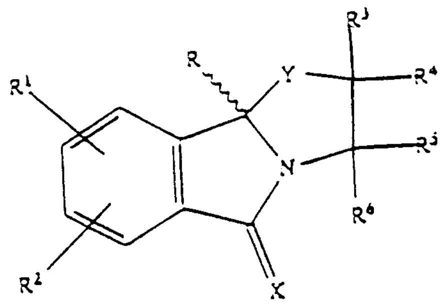

(I),

pour la préparation de médicaments ayant une activité antivirale, où

X peut être un atome d'oxygène ou de soufre, le groupe imino =NH ou un groupe =N-alkyl($C_1$-$C_5$)imino,

Y peut être un atome d'oxygène ou le groupe -$NR^7$, dans lequel $R^7$ représente un atome d'hydrogène ou un groupe alkyle en $C_1$-$C_6$ ou acyle en $C_1$-$C_6$,

R représente un atome d'hydrogène, un reste aliphatique saturé ou insaturé, à chaîne linéaire ou ramifiée, ayant 1-9 atomes de C, qui peut être substitué par un groupe phényle, ou un reste alcoxy($C_1$-$C_6$)alkyle($C_1$-$C_6$) ou alkyl($C_1$-$C_6$)mercapto-alkyle($C_1$-$C_6$), ou
un noyau phényle, qui peut être éventuellement substitué une ou plusieurs fois par un groupe alkyle en $C_1$-$C_6$, alcoxy en $C_1$-$C_6$, alkyl($C_1$-$C_6$)mercapto, alkyl($C_1$-$C_6$)sulfinyle, alkyl($C_1$-$C_6$)sulfonyle, alcényle en $C_2$-$C_6$, alcinyle en $C_2$-$C_6$, alcényloxy en $C_2$-$C_6$, alcényl($C_2$-$C_6$)mercapto, alcinyloxy en $C_2$-$C_6$ alcinyl($C_2$-$C_6$)mercapto, amino, alkyl($C_1$-$C_6$)amino, di[alkyl($C_1$-$C_6$)]amino, alkyl($C_1$-$C_6$)-carbonylamino, alkyl($C_1$-$C_6$)-aminocarbonyle, alcoxy($C_1$-$C_6$)carbonyle, hydroxy, benzyloxy, phénylmercapto, phényloxy, nitro, cyano, halogéno, trifluorométhyle, azido, formylamino, carboxy ou phényle, ou
un noyau carbocyclique mono-, bi- ou tricyclique ayant 7-15 atomes de carbone, ou un système hétèrocyclique mono-, bi- ou tricyclique ayant 5 ou 6 atomes par cycle, et pouvant contenir, par système cyclique, 1-4 ou 1-5 hétéroatomes, les hétéroatomes étant l'azote, le soufre ou l'oxygène,

$R^1$ représente un atome d'hydrogène, un reste aliphatique saturé ou insaturé, à chaîne linéaire ou ramifiée, ayant 1-6 atomes de C, ou un groupe alcoxy en $C_1$-$C_6$, alkyl($C_1$-$C_6$)mercapto, alkyl($C_1$-$C_6$)sulfinyle, alkyl($C_1$-$C_6$)sulfonyle, amino, alkyl($C_1$-$C_6$)amino, di[alkyl($C_1$-$C_6$)]amino, sulfonamido, alcoxy($C_1$-$C_6$)carbonyle, trifluorométhyle, carboxy, halogéno, hydroxy, nitro, cyano, azido, phényle ou benzyloxy,

$R^2$ a la même signification que $R^1$, les restes $R^1$ et $R^2$ pouvant être indépendamment identiques ou différents,

$R^3$ représente un atome d'hydrogène, un groupe alkyle en $C_1$-$C_6$, alcoxy en $C_1$-$C_6$, alkyl-($C_1$-$C_6$)mercapto, amino, alkyl($C_1$-$C_6$)amino, di[alkyl($C_1$-$C_6$)]amino, halogéno, cyano, hydroxy, carboxy, alcoxy($C_1$-$C_6$)carbonyle, aminocarbonyle, alkyl($C_1$-$C_6$)-aminocarbonyle ou di(alkyl($C_1$-$C_6$)]aminocarbonyle,

$R^4$, $R^5$, $R^6$ ont les mêmes significations que $R^3$, les restes $R^3$, $R^4$, $R^5$ et $R^6$ pouvant être indépendamment identiques ou différents,

ainsi que leurs tautomères, énantiomères, diastéréomères et sels physiologiquement acceptables.

2. Utilisation selon la revendication 1, caractérisée en ce que R représente un noyau carbocyclique ayant 7-15 atomes de C, choisi parmi les cycles naphtyle, anthracényle, phénanthrényle, fluorényle, indényle, indanyle, acénaphtylényle, norbornyle, adamantyle ou un groupe cycloalkyle en $C_3$-$C_7$ ou cycloalcényle en $C_5$-$C_8$, ces groupes pouvant être partiellement ou totalement hydrogénés.

3. Utilisation selon la revendication 1, caractérisée en ce que R représente un système mono-, bi- ou tricyclique, choisi parmi les groupes pyridinyle, pyrimidinyle, pyridazinyle, pyrazinyle, triazinyle, pyrollyle, pyrazollyle, imidazolyle, triazolyle, thiazolyle, oxazolyle, isoxazolyle, oxadiazolyle, furazanyle, furanyle, thiophényle, indolyle, quinoléinyle, isoquinoléinyle, coumaronyle, thionaphtényle, benzoxazolyle, benzothiazolyle, indazolyle, benzimidazolyle, benzotriazolyle, chroményle, phtalazinyle, quinazolinyle, quinoxalinyle, méthylènedioxybenzolyle, carbazolyle, acridinyle, phénoxazinyle, phénothiazinyle, phénazinyle ou purine, où les hétérocycles peuvent être partiellement ou totalement hydrogénés.

4. Utilisation selon la revendication 1, caractérisée en ce que

| | |
|---|---|
| X | peut être un atome d'oxygène ou de soufre, et |
| Y | peut être un atome d'oxygène ou le groupe $-NR^7$, dans lequel $R^7$ représente un atome d'hydrogène ou un groupe alkyle en $C_1$-$C_6$ ou acyle en $C_1$-$C_6$, et |
| R | représente un noyau phényle ou phényle substitué une ou deux fois par un groupe alkyle en $C_1$-$C_6$, alcoxy en $C_1$-$C_6$, alkyl($C_1$-$C_6$)mercapto, alkyl($C_1$-$C_6$)-sulfinyle, alkyl($C_1$-$C_6$)sulfonyle, alcényle en $C_2$-$C_6$, alcinyle en $C_2$-$C_6$, alcényloxy en $C_3$-$C_6$, alkyl($C_1$-$C_6$)amino, di[alkyl($C_1$-$C_6$)]amino, alkyl($C_1$-$C_6$)-carbonylamino, alkyl($C_1$-$C_6$)aminocarbonyle, alcoxy($C_1$-$C_6$)carbonyle, amino, hydroxy, nitro, azido, trifluorométhyle, cyano ou halogéno, ou<br>biphényle, naphtyle, anthracényle, indényle, fluorényle, acénaphtylényle, phénanthrényle, norbornyle, adamantyle, cycloalkyle en $C_3$-$C_6$,cycloalcényle en $C_5$-$C_8$, ou<br>pyrrolyle, amidazolyle, furanyle, thiophényle, pyridinyle, pyrimidinyle, thiazolyle, triazinyle, indolyle, quinoléinyle, isoquinoléinyle, coumaronyle, thionaphtényle, benzimidazolyle, quinazolinyle, méthylènedioxybenzolyle, éthylènedioxybenzolyle, carbazolyle, acridinyle ou phénothiazinyle, et |
| $R^1$ et $R^2$ | représentent un atome d'hydrogène, un groupe alkyle en $C_1$-$C_6$, alcényle en $C_2$-$C_6$, alcinyle en $C_2$-$C_6$, alcoxy en $C_1$-$C_6$, alkyl($C_1$-$C_6$)mercapto, alkyl($C_1$-$C_6$)-amino, alcoxy($C_1$-$C_6$)carbonyle, trifluorométhyle, amino, halogéno, hydroxy, cyano et azido, |
| $R^3$, $R^4$, $R^5$ et $R^6$ | représentent un atome d'hydrogène, un groupe alkyle en $C_1$-$C_6$,alcoxy en $C_1$-$C_6$, alkyl($C_1$-$C_6$)mercapto, carboxy, alcoxy($C_1$-$C_6$)carbonyle, halogéno, cyano et hydroxy. |

5. Dérivés d'oxazolo[2,3-a]-isoindol de formule générale Ia

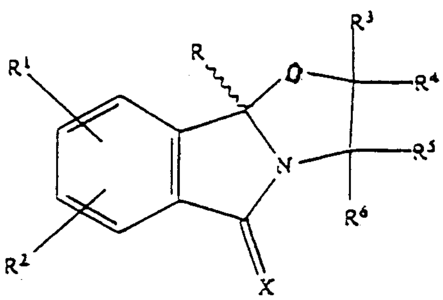

(Ia),

dans laquelle

| | |
|---|---|
| X | peut être un atome d'oxygène ou de soufre, le groupe imino =NH ou un groupe =N-alkyl($C_1$-$C_5$)imino, |
| R | représente un atome d'hydrogène, un reste aliphatique saturé ou insaturé, à chaîne linéaire ou ramifiée, ayant 1-9 atomes de 6, qui peut être substitué par un groupe phényle, ou un reste alcoxy($C_1$-$C_6$)alkyle($C_1$-$C_6$) ou alkyl($C_1$-$C_6$)mercapto-alkyle($C_1$-$C_6$), ou<br>un noyau phényle, qui peut être éventuellement substitué une ou plusieurs fois par un groupe alkyle en $C_1$-$C_6$, alcoxy en $C_1$-$C_6$, alkyl($C_1$-$C_6$)mercapto, alkyl($C_1$-$C_6$)sulfinyle$_1$ alkyl($C_1$-$C_6$)sulfonyle, alcényle en $C_2$-$C_6$, alcinyle en $C_2$-$C_6$, alcényloxy en $C_2$-$C_6$, alcényl($C_2$-$C_6$)mercapto, alcinyloxy en $C_2$-$C_6$, alcinyl($C_2$-$C_6$)mercapto, amino, alkyl-($C_1$-$C_6$)amino, di[alkyl($C_1$-$C_6$)]amino, alkyl($C_1$-$C_6$)carbonylamino,alkyl($C_1$-$C_6$)- |

aminocarbonyle, alcoxy($C_1$-$C_6$)carbonyle, hydroxy, benzyloxy, phénylmercapto, phényloxy, nitro, cyano, halogéno, trifluorométhyle, azido, formylamino, carboxy ou phényle, ou
un noyau carbocyclique mono-, bi- ou tricyclique ayant 7-15 atomes de carbone, ou un système hétérocyclique mono-, bi- ou tricyclique ayant 5 ou 6 atomes par cycle, et pouvant contenir, par système cyclique, 1-4 ou 1-5 hétéroatomes, les hétéroatomes étant l'azote, le soufre ou l'oxygène,

$R^1$ représente un reste aliphatique insaturé, à chaîne linéaire ou ramifiée, ayant jusqu'à 6 atomes de C, alkyl($C_1$-$C_6$)mercapto, alkyl($C_1$-$C_6$)sulfinyle, alkyl($C_1$-$C_6$)sulfonyle, alkyl($C_1$-$C_6$)amino, di[alkyl($C_1$-$C_6$)]amino, sulfonamido, alcoxy($C_1$-$C_6$)carbonyle, carboxy, hydroxy, cyano, azido, phényle ou benzyloxy,

$R^2$ représente un atome d'hydrogène ou a la même signification que $R^1$,

$R^3$ représente un atome d'hydrogène, un groupe alkyle en $C_1$-$C_6$, alcoxy en $C_1$-$C_6$, alkyl($C_1$-$C_6$)mercapto, amino, alkyl($C_1$-$C_6$)amino, di[alkyl($C_1$-$C_6$)]amino, halogéno, cyano, hydroxy, carboxy, alcoxy($C_1$-$C_6$)carbonyle, aminocarbonyle, alkyl($C_1$-$C_6$)-aminocarbonyle ou di[alkyl($C_1$-$C_6$)[aminocarbonyle,

$R^4$, $R^5$, $R^6$ ont les mêmes significations que $R^3$, les restes $R^3$, $R^4$, $R^5$ et $R^6$ pouvant être indépendamment identiques ou différents,

ainsi que leurs tautomères, énantiomères, diastéréomères et sels physiologiquement acceptables.

**6.** Dérivés de R- et S-oxazolo[2,3-a]isoindol et de R- et S-imidazo[2,1-a]isoindol de formule générale I

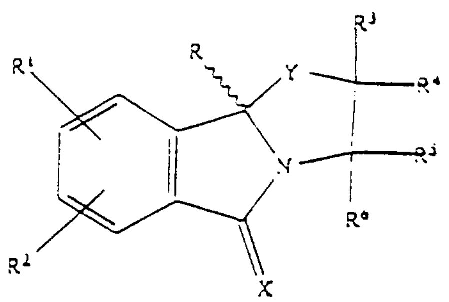

(I),

pour la préparation de médicaments ayant une activité antivirale, où

X peut être un atome d'oxygène ou de soufre, le groupe imino =NH ou un groupe =N-alkyl($C_1$-$C_5$)imino,

Y peut être un atome d'oxygène ou le groupe -$NR^7$, dans lequel $R^7$ représente un atome d'hydrogène ou un groupe alkyle en $C_1$-$C_6$ ou acyle en $C_1$-$C_6$,

R représente un atome d'hydrogène, un reste aliphatique saturé ou insaturé, à chaîne linéaire ou ramifiée, ayant 1-9 atomes de C, qui peut être substitué par un groupe phényle, ou un reste alcoxy($C_1$-$C_6$)alkyle($C_1$-$C_6$) ou alkyl($C_1$-$C_6$)mercapto-alkyle($C_1$-$C_6$), ou
un noyau phényle, qui peut être éventuellement substitué une ou plusieurs fois par un groupe alkyle en $C_1$-$C_6$, alcoxy en $C_1$-$C_6$, alkyl($C_1$-$C_6$)mercapto, alkyl($C_1$-$C_6$)sulfinyle, alkyl($C_1$-$C_6$)sulfonyle, alcényle en $C_2$-$C_6$, alcinyle en $C_2$-$C_6$, alcényloxy en $C_2$-$C_6$, alcényl($C_2$-$C_6$)mercapto, alcinyloxy en $C_2$-$C_6$, alcinyl($C_2$-$C_6$)mercapto, amino, alkyl($C_1$-$C_6$)amino, di[alkyl($C_1$-$C_6$)]amino, alkyl($C_1$-$C_6$)carbonylamino,alkyl($C_1$-$C_6$)-aminocarbonyle, alcoxy($C_1$-$C_6$)carbonyle, hydroxy, benzyloxy, phénylmercapto, phényloxy, nitro, cyano, halogéno, trifluorométhyle, azido, formylamino, carboxy ou phényle, ou
un noyau carbocyclique mono-, bi- ou tricyclique ayant 7-15 atomes de carbone, ou un système hétérocyclique mono-, bi- ou tricyclique ayant 5 ou 6 atomes par cycle, et pouvant contenir, par système cyclique, 1-4 ou 1-5 hétéroatomes, les hétéroatomes étant l'azote, le soufre ou l'oxygène,

$R^1$ représente un atome d'hydrogène, un reste aliphatique saturé ou insaturé, à chaîne linéaire ou ramifiée, ayant 1-6 atomes de C, ou un groupe alcoxy en $C_1$-$C_6$, alkyl($C_1$-$C_6$)mercapto, alkyl($C_1$-$C_6$)sulfinyle, alkyl($C_1$-$C_6$)sulfonyle, amino, alkyl($C_1$-$C_6$)amino, di[alkyl($C_1$-$C_6$)]amino, sulfonamido, alcoxy($C_1$-$C_6$)carbonyle, trifluorométhyle, carboxy,

halogéno, hydroxy, nitro, cyano, azido, phényle ou benzyloxy,

$R^2$ a la même signification que $R^1$, les restes $R^1$ et $R^2$ pouvant être indépendamment identiques ou différents,

$R^3$ représente un atome d'hydrogène, un groupe alkyle en $C_1$-$C_6$, alcoxy en $C_1$-$C_6$, alkyl-($C_1$-$6_6$)mercapto, amino, alkyl($C_1$-$C_6$)amino, di[alkyl($C_1$-$C_6$)]amino, halogéno, cyano, hydroxy, carboxy, alcoxy($C_1$-$C_6$)carbonyle aminocarbonyle, alkyl($C_1$-$C_6$)-aminocarbonyle ou di[alkyl($C_1$-$C_6$)]aminocarbonyle

$R^4$, $R^5$, $R^6$ ont les mêmes significations que $R^3$, les restes $R^3$, $R^4$, $R^5$ et $R^6$ pouvant être indépendamment identiques ou différents,

ainsi que leurs tautomères, diastéréomères et sels physiologiquement acceptables.

**7.** Médicament, contenant au moins un composé de formule I ou Ia selon la revendication 5 ou 6, en plus d'adjuvants et véhicules pharmaceutiquement acceptables.

**8.** Utilisation de composés de formule I ou Ia selon la revendication 5 ou 6, pour la préparation de médicaments destinés au traitement d'infections virales ou rétrovirales, ou de maladies provoquées par ces infections, telles que le SIDA ou l'ARC.

**9.** Procédé pour la préparation de médicaments contenant au moins un composé de formule I ou Ia selon la revendication 5 ou 6, en plus d'adjuvants et véhicules pharmaceutiquement acceptables, caractérisé en ce que l'on mélange un composé de formule I ou Ia avec les véhicules et adjuvants et on le transforme en la forme galénique appropriée.